# EUROPEAN PATENT APPLICATION

(11) **EP 4 597 509 A1**
(43) Date of publication of application: **06.08.2025**
(21) Application number: 24214171.1
(22) Date of filing: 20.11.2024
(51) Int. Cl.: G16H 40/20, G06Q 10/02, G06Q 10/1093

(54) **METHOD AND APPARATUS FOR ADJUSTING RESERVATION CAPACITY ACCORDING TO NO-SHOW RATE BASED ON PRESCRIPTION INFORMATION OF PATIENT**

(30) Priority: 05.02.2024 KR 20240017662
(71) Applicant: Samsung Life Public Welfare Foundation, Seoul 04348 (KR)
(72) Inventor: LEE, Jun Haeng, 06351 Seoul (KR); LEE, Kwang Hyuck, 06351 Seoul (KR); SEOL, Jeong Sook, 06351 Seoul (KR); KIM, Se Mi, 06351 Seoul (KR); NA, Eun Mi, 06351 Seoul (KR); KIM, Hyo Won, 06351 Seoul (KR); LEE, Jung Hee, 06351 Seoul (KR); KIM, Sun Ok, 06351 Seoul (KR); SEO, Jong Hee, 06351 Seoul (KR); LEE, Ji Min, 06351 Seoul (KR); KO, Mun Jeong, 06351 Seoul (KR); OH, Sei Yeul, 06351 Seoul (KR); LIM, Do Hoon, 06351 Seoul (KR); LEE, Ihn Seon, 06351 Seoul (KR); YOON, So Young, 06351 Seoul (KR); KIM, Keun Hwa, 06351 Seoul (KR); JEONG, Seok Doo, 06351 Seoul (KR); CHOI, Jae Kyun, 06351 Seoul (KR); MOON, Jung Hwan, 06351 Seoul (KR); RHEE, Poong Lyul, 06351 Seoul (KR)
(74) Representative: Weickmann & Weickmann PartmbB

(57) **Abstract**

According to one embodiment, a reservation capacity adjustment apparatus performs: acquiring prescription information of a patient and reservation information of the patient; filtering reservation information held based on at least one piece of first information among the prescription information, and calculating a no-show rate of the reservation information; and adjusting a reservation capacity for the first information based on the no-show rate.

## Description

### BACKGROUND OF THE INVENTION

### 1. Field of the Invention

The present invention relates to a technology for effectively adjusting a reservation capacity of a hospital by using a no-show rate of a reservation, which is calculated based on prescription information and reservation information of a patient.

### 2. Description of the Related Art

With the development of medical technologies, medical choices of customers are expanding, and medical services are becoming more customer-centered, so that there is a need for an efficient reservation operation system in hospitals so that the customers may receive treatments conveniently and rapidly at hospitals.

In particular, the hospitals have limited medical resources such as the number of doctors, consultation times assigned to the doctors, the number of operating rooms, and the number of surgical equipment, so that a reservation capacity that a hospital may accept is limited.

Accordingly, when a no-show reservation occurs in the limited reservation capacity, the customers will have to wait longer to make a surgery reservation or receive a specific procedure, and the hospitals will have difficulties in providing efficient services because of human, material, and time losses that may not be utilized due to no-show reservations.

Accordingly, there is a need for a reservation operation system capable of flexibly adjusting a reservation capacity of a hospital in consideration of a reservation no-show rate according to various criteria.

### SUMMARY OF THE INVENTION

An object of the present invention is to provide a technology for effectively adjusting a reservation capacity by using a no-show rate of a reservation, which is calculated based on prescription information and reservation information of a patient.

Meanwhile, technical objects of the present invention are not limited to the technical objects described above, and other technical objects that are not described above will be clearly understood by those skilled in the art from the description below.

According to one embodiment, there is provided a method performed by a reservation capacity adjustment apparatus operated by a processor, the method including: acquiring prescription information and reservation information of a patient; filtering reservation information held based on at least one piece of first information among the prescription information, and calculating a no-show rate of the reservation information; and adjusting a reservation capacity for the first information based on the no-show rate.

In addition, the prescription information may include information on a doctor in charge according to prescription, a type of procedure according to the prescription, and a period from a date on which the prescription is issued to a date on which the procedure is reserved.

In addition, the calculating of the no-show rate may include calculating the no-show rate by aggregating reservation information having a no-show reservation among reservation information filtered based on the first information during a preset period.

In addition, the adjusting of the reservation capacity may include setting an updated first reservation capacity by adding a ratio of the no-show rate for a first reservation capacity that is preset based on the first information to an existing first reservation capacity.

In addition, the first information may include information on whether the doctor in charge of the prescription is an assigned doctor or an unassigned doctor, and the setting of the updated first reservation capacity may include applying different weights to the ratio according to whether the doctor in charge of the prescription is the assigned doctor or the unassigned doctor, and adding the ratio to the existing reservation capacity, when the ratio of the no-show rate of the first reservation capacity that is preset based on the first information is added to the existing reservation capacity.

In addition, the calculating of the no-show rate may include calculating the no-show rate by aggregating the reservation information having the no-show reservation among reservation information filtered based on reservation information in which the period from the date on which the prescription is issued to the date on which the procedure is reserved falls within a same range, and the adjusting of the reservation capacity may include setting an updated first reservation capacity by adding a ratio of the no-show rate for a first reservation capacity that is preset based on the period from the date on which the prescription is issued to the date on which the procedure is reserved to an existing first reservation capacity.

In addition, the reservation information in which the period falls within the same range may include reservation information in which information on whether the doctor in charge of the prescription is an assigned doctor or an unassigned doctor and the type of procedure according to the prescription is same.

According to one embodiment, there is provided a reservation capacity adjustment apparatus comprising: a memory including an instruction; and a processor for performing a predetermined operation based on the instruction, wherein the operation of the processor includes: acquiring prescription information and reservation information of a patient; filtering reservation information held based on at least one piece of first information among the prescription information, and calculating a no-show rate of the reservation information; and adjusting a reservation capacity for the first information based on the no-show rate.

In addition, the prescription information may include information on a doctor in charge according to prescription, a type of procedure according to the prescription, and a period from a date on which the prescription is issued to a date on which the procedure is reserved.

In addition, the calculating of the no-show rate may include calculating the no-show rate by aggregating reservation information having a no-show reservation among reservation information filtered based on the first information during a preset period.

In addition, the adjusting of the reservation capacity may include setting an updated first reservation capacity by adding a ratio of the no-show rate for a first reservation capacity that is preset based on the first information to an existing first reservation capacity.

In addition, the first information may include information on whether the doctor in charge of the prescription is an assigned doctor or an unassigned doctor, and the setting of the updated first reservation capacity may include applying different weights to the ratio according to whether the doctor in charge of the prescription is the assigned doctor or the unassigned doctor, and adding the ratio to the existing reservation capacity, when the ratio of the no-show rate of the first reservation capacity that is preset based on the first information is added to the existing reservation capacity.

In addition, the calculating of the no-show rate may include calculating the no-show rate by aggregating the reservation information having the no-show reservation among reservation information filtered based on reservation information in which the period from the date on which the prescription is issued to the date on which the procedure is reserved falls within a same range, and the adjusting of the reservation capacity may include setting an updated first reservation capacity by adding a ratio of the no-show rate for a first reservation capacity that is preset based on the period from the date on which the prescription is issued to the date on which the procedure is reserved to an existing first reservation capacity.

In addition, the reservation information in which the period falls within the same range may include reservation information in which information on whether the doctor in charge of the prescription is an assigned doctor or an unassigned doctor and the type of procedure according to the prescription is same.

According to one embodiment, there is provided a computer-readable recording medium for storing a computer program, wherein the computer-readable recording medium includes an instruction for allowing a processor to perform a method including: acquiring prescription information and reservation information of a patient; filtering reservation information held based on at least one piece of first information among the prescription information, and calculating a no-show rate of the reservation information; and adjusting a reservation capacity for the first information based on the no-show rate.

According to the present invention, a reservation capacity can be flexibly adjusted by calculating a no-show rate of a reservation based on various indicators associated with prescription information of a patient, so that customers have to wait shorter to make a surgery reservation or receive a specific procedure, and hospitals can provide more efficient medical services due to reduction in human, material, and time losses caused by no-show reservations.

Meanwhile, effects of the present invention are not limited to the effects described above, and other technical effects that are not described above will be clearly understood by those skilled in the art from the description below.

### BRIEF DESCRIPTION OF THE DRAWINGS

FIG. 1 is a view showing a configuration of a reservation capacity adjustment apparatus according to one embodiment.
FIG. 2 is a flowchart showing steps of operations performed by a reservation capacity adjustment apparatus according to one embodiment.
FIG. 3 is an exemplary view for describing an operation of filtering reservation information according to first information and calculating a no-show rate, which is performed by the reservation capacity adjustment apparatus according to one embodiment.
FIG. 4 is an exemplary view for describing an operation of increasing an existing first reservation capacity based on a no-show rate according to first information, which is performed by the reservation capacity adjustment apparatus according to one embodiment.
FIG. 5 is an exemplary view for describing an operation of increasing an existing first reservation capacity based on a no-show rate when information according to first information corresponds to an unassigned doctor, which is performed by the reservation capacity adjustment apparatus according to one embodiment.
FIG. 6 is an exemplary view for describing an operation of increasing an existing first reservation capacity based on reservation information in which a period from a date on which prescription is issued to a date on which a procedure is reserved falls within the same range, which is performed by the reservation capacity adjustment apparatus according to one embodiment.

### DETAILED DESCRIPTION OF THE INVENTION

Hereinafter, embodiments of the present invention will be described with reference to the drawings. In the following description, descriptions of a well-known technical configuration in relation to a lead implantation system for a deep brain stimulator will be omitted. For example, descriptions of the configuration/structure/method of a device or system commonly used in deep brain stimulation, such as the structure of an implantable pulse generator, a connection structure/method of the implantable pulse generator and a lead, and a process for transmitting and receiving electrical signals measured through the lead with an external device, will be omitted. Even if these descriptions are omitted, one of ordinary skill in the art will be able to easily understand the characteristic configuration of the present invention through the following description.

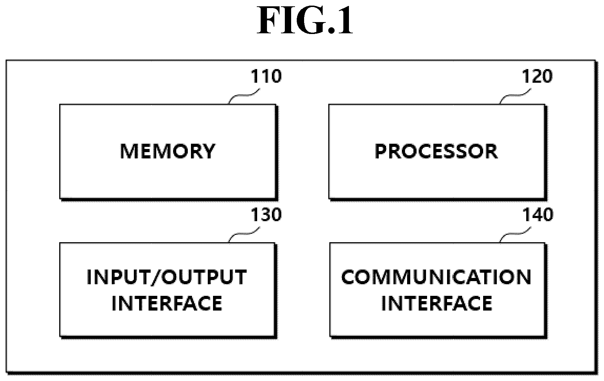

FIG. 1 is a view showing a configuration of a reservation capacity adjustment apparatus 100 (hereinafter referred to as an "apparatus 100") according to one embodiment.

Referring to FIG. 1, according to one embodiment, an apparatus 100 may include a memory 110, a processor 120, an input/output interface 130, and a communication interface 140.

The memory 110 may store data acquired from an external device or data generated by the memory 110. The memory 110 may include a volatile memory and a nonvolatile memory. The memory 110 may store instructions that may perform operations of the processor 120. In addition, the memory 110 may store prescription information of a patient and reservation information of the patient, which will be described below.

The processor 120 may refer to an arithmetic device that controls an overall operation. The processor 120 may execute the instructions stored in the memory 110. Operations of the apparatus 100 according to an embodiment of the present disclosure may be understood as operations performed by the processor 120.

The input/output interface 130 may include a hardware interface or a software interface, which inputs or outputs information.

The communication interface 140 may enable information to be transmitted and received through a communication network. To this end, the communication interface 140 may include a wireless communication module or a wired communication module.

The apparatus 100 may be implemented as various apparatuses capable of performing calculations through the processor 120, and transmitting and receiving information through a network. For example, the apparatus 100 may be implemented in the form of a server, a computer device, a portable communication device, a smartphone, a portable multimedia device, a laptop computer, a tablet PC, and the like, but is not limited to these examples.

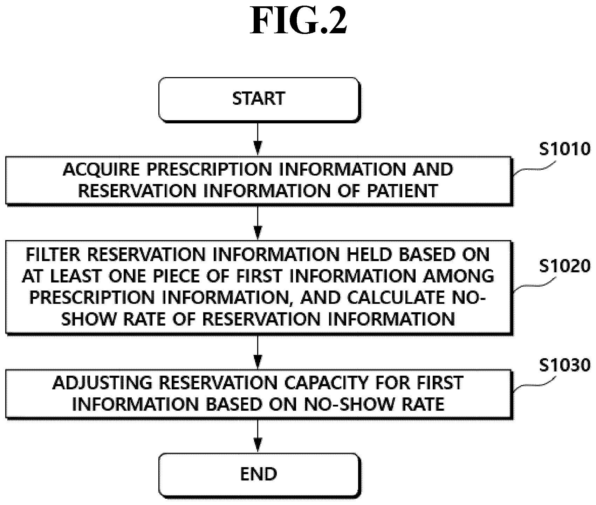

FIG. 2 is a flowchart showing operations performed by an apparatus 100 according to one embodiment. Operations of the apparatus 100 according to an embodiment of FIG. 2 may be understood as operations performed by the processor 120. Each step disclosed in FIG. 2 is only an exemplary embodiment for achieving the object of the present invention, some steps may be added or removed as needed, and one step may be included in another step and performed. An order of the operations disclosed in FIG. 2 is only an order arranged for convenience of understanding, so that the order is not limited to a chronological order, and the order may be changed differently and operated according to a selection of a designer.

Referring to FIG. 2, in a step S1010, the apparatus 100 may acquire prescription information and reservation information of a patient. For example, the apparatus 100 may store the prescription information and the reservation information of the patient in the memory 110. For example, the apparatus 100 may acquire the prescription information and the reservation information of the patient from a database associated with reservation management of a hospital.

For example, the prescription information may include information on a doctor in charge according to prescription, a type of procedure, and a period from a date on which the prescription is issued to a date on which the procedure is reserved.

For example, the reservation information may include information on a reservation date on which the procedure of the patient is to be performed.

The apparatus 100 may manage the prescription information and the reservation information by mapping the prescription information and the reservation information based on a specific patient. Accordingly, the apparatus 100 may simultaneously retrieve the prescription information and the reservation information of the specific patient.

In a step S1020, the apparatus 100 may filter reservation information held based on at least one piece of first information among the prescription information (e.g., a doctor in charge according to prescription, a type of procedure according to the prescription, a period from a date on which the prescription is issued to a date on which the procedure is reserved, etc.), and calculate a no-show rate of the filtered reservation information. For example, when the first information is 'Doctor A', the reservation information assigned to 'Doctor A' may be filtered for a preset period. For example, when the first information is 'Procedure B', the reservation information of a patient who is reserved to receive 'Procedure B' may be filtered for a preset period. For example, when the first information is "January 1, 2023 - December 31, 2023", the reservation information of a patient who made reservations during "January 1, 2023 - December 31, 2023" may be filtered. Two or more pieces of the first information may be selected simultaneously according to a selection of a manager.

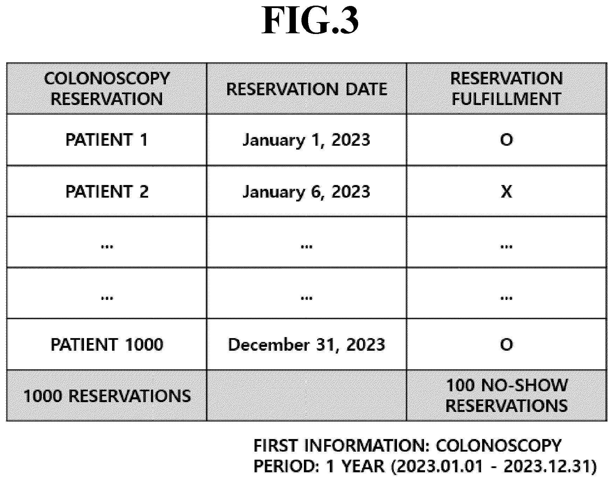

FIG. 3 is an exemplary view for describing an operation of filtering reservation information according to first information and calculating a no-show rate, which is performed by the apparatus 100 according to one embodiment.

Referring to FIG. 3, when assuming a case in which the first information is "colonoscopy", the apparatus 100 may filter all reservation information that is reserved based on "colonoscopy" during a preset period (e.g., 1 year). The apparatus 100 may calculate the no-show rate by aggregating reservation information having no-show among the filtered reservation information.

For example, when all reservation information reserved at the hospital for the year 2023 based on "colonoscopy" is 1,000, and 100 among 1,000 have a no-show reservation, the apparatus 100 may calculate that the reservation no-show rate retrieved based on "colonoscopy" is 10%.

In a step S1030, the apparatus 100 may adjust a reservation capacity for the first information based on the calculated no-show rate.

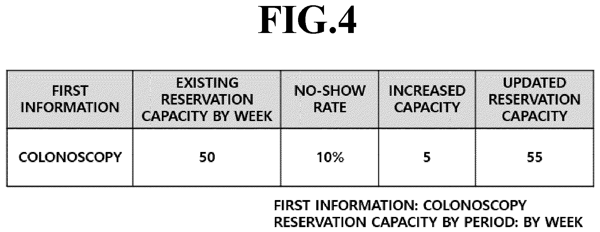

For example, the apparatus 100 may set an updated first reservation capacity by adding a ratio of the no-show rate calculated in the step S1020 to a first reservation capacity that is preset based on the first information. A specific example of the above configuration is shown in FIG. 4 below.

FIG. 4 is an exemplary view for describing an operation of increasing an existing first reservation capacity based on a no-show rate according to first information, which is performed by the apparatus 100 according to one embodiment.

Referring to FIG. 4, when a case in which the first information is "colonoscopy" is described with the previous example, it may be assumed that the number of preset first reservation capacities that are preset so that "colonoscopy" procedure reservations may be assigned during a preset period (e.g., one week) is 50. In this case, the apparatus 100 may additionally set 5 corresponding to a no-show rate of 10% to the existing reservation capacity of 50, thereby updating the first reservation capacity that may be assigned to a "colonoscopy" procedure for one week to 55.

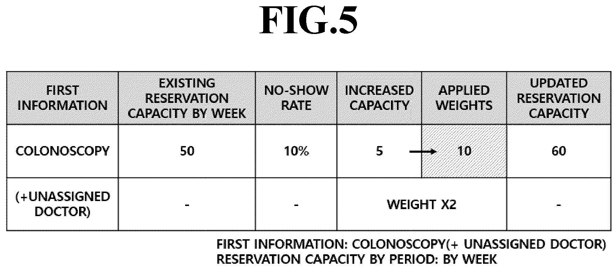

In addition, as an additional embodiment, the first information may include information on whether the doctor in charge is an assigned doctor or an unassigned doctor, and the apparatus 100 may additionally apply different weights to the ratio according to whether the doctor in charge is the assigned doctor or the unassigned doctor when the ratio of the no-show rate of the first reservation capacity that is preset based on the first information in the step S1030 is added to the existing reservation capacity. A specific example of the above configuration is shown in FIG. 5 below.

FIG. 5 is an exemplary view for describing an operation of increasing an existing first reservation capacity based on a no-show rate when information according to first information corresponds to an unassigned doctor, which is performed by the apparatus 100 according to one embodiment.

Referring to FIG. 5, a case in which the first information is "colonoscopy", the number of first reservation capacities that are preset so that "colonoscopy" procedure reservations may be assigned during a preset period (e.g., one week) is 50, and the no-show rate is assumed to be 10% is described with the previous example. In an example of FIG. 5, it may be assumed that the weight is "0" when the doctor in charge is the assigned doctor, and the weight is "2x" when the doctor in charge is the unassigned doctor. In this case, since the first information is "colonoscopy" + "unassigned doctor", when the weight of 2x is applied to 5 that corresponds to 10% of the existing one-week reservation capacity of 50, it may be weighted to 5 x 2x = 10. Accordingly, the apparatus 100 may update the first reservation capacity that may be assigned to "colonoscopy" procedures for one week to 60.

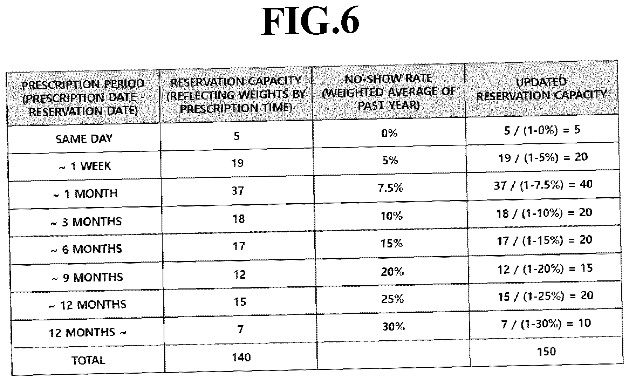

FIG. 6 is an exemplary view for describing an operation of increasing an existing first reservation capacity based on reservation information in which a period from a date on which prescription is issued to a date on which a procedure is reserved falls within the same range, which is performed by the apparatus 100 according to one embodiment.

In detail, FIG. 6 is a table obtained by filtering reservation information according to a period range from the date on which the prescription is issued to the date on which the procedure is reserved for reservation information held based on the same prescription information (e.g., assignment state: unassigned doctor, procedure type: esophagogastroduodenoscopy) as of a current date.

Referring to FIG. 6, the apparatus 100 may calculate the no-show rate by filtering reservation information having a no-show reservation among reservation information filtered based on reservation information in which the period from the date on which the prescription is issued to the date on which the procedure is reserved falls within the same range (e.g., same day, within 1 week, within 1 month, within 3 months, etc.) in the filtering operation of the step S1020. In this case, the reservation information in which the period falls within the same range may include only reservation information in which the same target to be compared, such as information on whether the doctor in charge of the prescription is an assigned doctor or an unassigned doctor and the type of procedure according to the prescription is the same.

Accordingly, the apparatus 100 may aggregate the no-show rate by performing filtering according to the period from the date on which the prescription is issued to the date on which the procedure is reserved based on the reservation information within the past year. For example, the apparatus 100 may aggregate the no-show rate based on a simple average or a weighted average according to settings of the manager. For example, when the apparatus 100 uses the weighted average, the no-show rate may be obtained by adding a weight according to the period within which the reservation information falls among the reservation information within the past year. For example, a weight of 0.4 may be set to the reservation information of the past 3 months, a weight of 0.3 may be set to the reservation information of the past 3 to 6 months, a weight of 0.2 may be set to the reservation information of the past 6 to 9 months, and a weight of 0.1 may be set to the reservation information of the past 9 to 12 months, so that the no-show rate may be calculated based on the weighted average obtained by multiplying the number of no-shows issued during the period by the weight and calculating the average.

Thereafter, the apparatus 100 may additionally reflect a reservation capacity acceptable on a current or future reservation date based on a pattern of the no-show rate calculated in the operation of the step S1020 described above. For example, the apparatus 100 may set an updated first reservation capacity by adding a ratio of the aggregated no-show rate for a first reservation capacity that is preset based on the period from the date on which the prescription is issued to the date on which the procedure is reserved to an existing first reservation capacity, such as "first reservation capacity/(1-no-show rate%)=updated first reservation capacity".

As an additional embodiment, the apparatus 100 may provide the following reservation schedule management function in relation to the setting of the reservation capacity.

For example, detailed information corresponding to requirements for each medical practitioner may be generated for customized reservations for each medical practitioner.

For example, the reservation capacity may be set as the number of people or a time interval.

For example, an available examination day of the week and an available examination room may be set to perform a function that is necessary to create a schedule that matches a procedure schedule of a medical practitioner.

For example, an available examination medical department and an available examination doctor may be set to prevent reservation errors by setting a medical department and a prescription doctor, which may be reserved, to the medical practitioner.

For example, an available examination item and a required examination time may be set in order to prevent reservation errors by setting an examination item that may be reserved to each detailed information for each medical practitioner.

For example, simultaneous examination reservations may be enabled so that it may be checked whether the same patient may make two or more examination reservations at the same time.

For example, patients may be set to be distinguished in order to retrieve endoscopy reservation schedules that are suitable for outpatients/emergency patients/inpatients/discharge patients.

For example, integrated reservations may be set in order to control an integrated reservation availability state, a time limit, and a person having authority.

For example, reservation changes may be set in order to prevent errors by restricting the ability to maintain/enable existing information when changing reservations.

For example, examinations may be directly set in order to control whether the prescription doctor and an execution doctor are the same.

For example, a reservation rate indicator of an empty reservation capacity may be automatically converted into a high value (e.g., 95% or more) in order to efficiently manage the reservation capacity by automatically converting the empty reservation capacity that has not been reserved at a specific time.

For example, different colors may be assigned for each detailed information of the reservation capacity, so that a color may be displayed on reservation capacity information to allow the reservation capacity to be intuitively viewed.

For example, functions of reserving and modifying endoscopy patients, changing patient reservations when the procedure is absent, and recognizing an endoscopy reservation status of the medical practitioner may be provided.

For example, a function of retrieving the reservation capacity (e.g., for each examination room, or for each examination doctor) on a calendar and a time status board may be provided.

For example, a function of registering a reservation schedule by performing drag-and-drop from a patient list to an empty reservation capacity may be provided.

For example, a function of displaying an absence date on the calendar when the medical practitioner is absent on a specific date may be provided.

For example, a function of changing a reservation schedule of the patient may be provided by moving a reservation change target.

For example, when more reservations are required than an actual reservation capacity depending on operating situations of the hospital, an arbitrary reservation capacity may be generated.

For example, a function of recognizing an endoscopic examination reservation status of the medical practitioner may be provided.

For example, a function of recognizing a reservation capacity and a patient examination status of an examination room for each week may be provided.

For example, a function of schedule creation, copying, deletion, examination room movement, and reservation failure, which are related to reservations, may be provided.

For example, a function of managing procedures of an endoscopy examination room for each period may be provided.

For example, a pop-up function that is checked when entering the absence date or when entering multiple entries may be provided.

For example, a function of displaying the absence date in a different background color may be provided.

For example, convenience of input may be provided by copying schedules and creating and uploading templates.

For example, a function of changing a state into temporary/confirmed when entering reservation information may be provided.

For example, a function of setting an absence period and blocking a reservation schedule of the medical practitioner may be provided.

For example, a function of blocking a reservation schedule for each day of the week may be provided.

For example, a function of registering absence by setting a repeating week or day of the week may be provided.

For example, a history management function may be provided through a registration date, a modification date, and a deletion date.

For example, a function of changing a required examination item time for each detailed information by detecting a required time entered in a clinical opinion when prescribing examination of a patient may be provided.

For example, a function of collectively retrieving detailed information in which an examination item is registered and the required time may be provided.

For example, as a function of additional information and reference contents for reserved patients, a function of displaying an execution doctor and a nurse in charge for each time zone, a function of managing entrance to an examination room as an examination room holding function, a function of displaying an exit time by processing a background color of a recovery room entrance cell, and a function of displaying whether a transfer agent is called may be provided.

For example, a function of retrieving a no-show rate based on the detailed information for each medical practitioner, and retrieving a reservation rate/no-show rate for each number of reservation execution cases and a reservation capacity based on a reservation date so as to utilize the reservation capacity as a management index may be provided.

For example, as a function of reserving an examination room for each location and retrieving a progress status, a function of recognizing an examination progress state of a patient based on an examination room, a function of retrieving a reservation patient status for each examination room, and a function of retrieving an examination status, a reservation, and an execution case status for each time zone may be provided.

As an additional embodiment, the apparatus 100 may provide a template function association with the setting of the reservation capacity.

For example, the apparatus 100 may provide: a function of managing a reservation CAPA of an examination room for a specific day of the week and a specific period as a template; a function of creating a template and collectively generating a reservation capacity by reflecting the special nature of examination of the assigned doctor (in cases where a specific medical practitioner is required to perform a specific examination) and the unassigned doctor (in cases where all medical practitioners may perform a specific examination); a function of selecting a use state and an order of a template, scale adjustment, a start/end time, and a day of the week; and a function of collectively retrieving detailed information of the template through a detailed information list for each template.

According to the embodiments of the present invention described above, a reservation capacity may be flexibly adjusted by calculating a no-show rate of a reservation based on various indicators associated with prescription information of a patient, so that customers may have to wait shorter to make a surgery reservation or receive a specific procedure, and hospitals may provide more efficient medical services due to reduction in human, material, and time losses caused by no-show reservations.

The embodiments described above may be implemented by hardware components, software components, and/or any combination thereof. For example, the devices, the methods, and components described in the embodiments may be implemented by using general-purpose computers or special-purpose computers, such as a processor, a controller, an arithmetic logic unit (ALU), a digital signal processor, a microcomputer, a field programmable gate array (FPGA), a programmable logic unit (PLU), a microprocessor, or any other devices which may execute and respond to instructions. A processing apparatus may execute an operating system (OS) and a software application executed in the OS. Also, the processing apparatus may access, store, operate, process, and generate data in response to the execution of software. For convenience of understanding, it may be described that one processing apparatus is used. However, one of ordinary skill in the art will understand that the processing apparatus may include a plurality of processing elements and/or various types of processing elements. For example, the processing apparatus may include a plurality of processors or a processor and a controller. Also, other processing configurations, such as a parallel processor, are also possible.

The software may include computer programs, code, instructions, or any combination thereof, and may construct the processing apparatus for desired operations or may independently or collectively command the processing apparatus. In order to be interpreted by the processing apparatus or to provide commands or data to the processing apparatus, the software and/or data may be permanently or temporarily embodied in any types of machines, components, physical devices, virtual equipment, computer storage mediums, or transmitted signal waves. The software may be distributed over network coupled computer systems so that it may be stored and executed in a distributed fashion. The software and/or data may be recorded in a computer-readable recording medium.

A method according to an embodiment may be implemented as program instructions that can be executed by various computer devices, and recorded on a computer-readable recording medium. The computer-readable recording medium may include program instructions, data files, data structures or a combination thereof. Program instructions recorded on the medium may be particularly designed and structured for embodiments or available to one of ordinary skill in a field of computer software. Examples of the computer-readable recording medium include magnetic media, such as a hard disc, a floppy disc, and magnetic tape; optical media, such as a compact disc-read only memory (CD-ROM) and a digital versatile disc (DVD); magnetooptical media, such as floptical discs; and hardware devices specially configured to store and execute program instructions, such as ROM, random-access memory (RAM), a flash memory, etc. Program instructions may include, for example, high-level language code that can be executed by a computer using an interpreter, as well as machine language code made by a complier.

In concluding the detailed description, those skilled in the art will appreciate that many variations and modifications may be made to the preferred embodiments without substantially departing from the principles of the present invention. Therefore, the disclosed preferred embodiments of the invention are used in a generic and descriptive sense only and not for purposes of limitation.

According to one embodiment, a reservation capacity adjustment apparatus performs: acquiring prescription information of a patient and reservation information of the patient; filtering reservation information held based on at least one piece of first information among the prescription information, and calculating a no-show rate of the reservation information; and adjusting a reservation capacity for the first information based on the no-show rate.

## Claims

1. A method performed by a reservation capacity adjustment apparatus operated by a processor, the method comprising:
acquiring prescription information and reservation information of a patient;
filtering reservation information held based on at least one piece of first information among the prescription information, and calculating a no-show rate of the reservation information; and
adjusting a reservation capacity for the first information based on the no-show rate.

2. The method of claim 1, wherein the prescription information includes information on a doctor in charge according to prescription, a type of procedure according to the prescription, and a period from a date on which the prescription is issued to a date on which the procedure is reserved.

3. The method of claims 1 or 2, wherein the calculating of the no-show rate includes calculating the no-show rate by aggregating reservation information having a no-show reservation among reservation information filtered based on the first information during a preset period.

4. The method of any of the preceding claims, wherein the adjusting of the reservation capacity includes setting an updated first reservation capacity by adding a ratio of the no-show rate for a first reservation capacity that is preset based on the first information to an existing first reservation capacity.

5. The method of any of the preceding claims, wherein the first information includes information on whether the doctor in charge of the prescription is an assigned doctor or an unassigned doctor, and
the setting of the updated first reservation capacity includes applying different weights to the ratio according to whether the doctor in charge of the prescription is the assigned doctor or the unassigned doctor, and adding the ratio to the existing reservation capacity, when the ratio of the no-show rate of the first reservation capacity that is preset based on the first information is added to the existing reservation capacity.

6. The method of any of the preceding claims, wherein the calculating of the no-show rate includes calculating the no-show rate by aggregating the reservation information having the no-show reservation among reservation information filtered based on reservation information in which the period from the date on which the prescription is issued to the date on which the procedure is reserved falls within a same range, and
the adjusting of the reservation capacity includes setting an updated first reservation capacity by adding a ratio of the no-show rate for a first reservation capacity that is preset based on the period from the date on which the prescription is issued to the date on which the procedure is reserved to an existing first reservation capacity.

7. The method of any of the preceding claims, wherein the reservation information in which the period falls within the same range includes reservation information in which information on whether the doctor in charge of the prescription is an assigned doctor or an unassigned doctor and the type of procedure according to the prescription is the same.

8. A reservation capacity adjustment apparatus comprising:
a memory including an instruction; and
a processor for performing a predetermined operation based on the instruction,
wherein the operation of the processor includes:
acquiring prescription information and reservation information of a patient;
filtering reservation information held based on at least one piece of first information among the prescription information, and calculating a no-show rate of the reservation information; and
adjusting a reservation capacity for the first information based on the no-show rate.

9. The reservation capacity adjustment apparatus of claim 8, wherein the prescription information includes information on a doctor in charge according to prescription, a type of procedure according to the prescription, and a period from a date on which the prescription is issued to a date on which the procedure is reserved.

10. The reservation capacity adjustment apparatus of claim 8 or 9, wherein the calculating of the no-show rate includes calculating the no-show rate by aggregating reservation information having a no-show reservation among reservation information filtered based on the first information during a preset period.

11. The reservation capacity adjustment apparatus of any of claims 8-10, wherein the adjusting of the reservation capacity includes setting an updated first reservation capacity by adding a ratio of the no-show rate for a first reservation capacity that is preset based on the first information to an existing first reservation capacity.

12. The reservation capacity adjustment apparatus of claim 11, wherein the first information includes information on whether the doctor in charge of the prescription is an assigned doctor or an unassigned doctor, and
the setting of the updated first reservation capacity includes applying different weights to the ratio according to whether the doctor in charge of the prescription is the assigned doctor or the unassigned doctor, and adding the ratio to the existing reservation capacity, when the ratio of the no-show rate of the first reservation capacity that is preset based on the first information is added to the existing reservation capacity.

13. The reservation capacity adjustment apparatus of any of claims 8-12, wherein the calculating of the no-show rate includes calculating the no-show rate by aggregating the reservation information having the no-show reservation among reservation information filtered based on reservation information in which the period from the date on which the prescription is issued to the date on which the procedure is reserved falls within a same range, and
the adjusting of the reservation capacity includes setting an updated first reservation capacity by adding a ratio of the no-show rate for a first reservation capacity that is preset based on the period from the date on which the prescription is issued to the date on which the procedure is reserved to an existing first reservation capacity.

14. The reservation capacity adjustment apparatus of any of claims 8-13, wherein the reservation information in which the period falls within the same range includes reservation information in which information on whether the doctor in charge of the prescription is an assigned doctor or an unassigned doctor and the type of procedure according to the prescription is same.

15. A computer-readable recording medium for storing a computer program, wherein the computer-readable recording medium includes an instruction for allowing a processor to perform a method including:
acquiring prescription information and reservation information of a patient;
filtering reservation information held based on at least one piece of first information among the prescription information, and calculating a no-show rate of the reservation information; and
adjusting a reservation capacity for the first information based on the no-show rate.
